# EUROPEAN PATENT SPECIFICATION

(11) **EP 0 465 449 B1**
(45) Date of publication and mention of the grant of the patent: **26.11.1997**
(21) Application number: 91850181.8
(22) Date of filing: 28.06.1991
(51) Int. Cl.: A61B 17/39

(54) **Tissue dissipative recanalization catheter.**
Katheter zur dissipativen Rekanalisierung von Gewebe
Cathéter pour la recanalisation dissipative dans un tissu

(30) Priority: 02.07.1990 US 547473
(43) Date of publication of application: 08.01.1992
(73) Proprietor: Heart Technology, Inc., Bellevue, Washington 98005 (US)
(72) Inventor: Auth, David C., Kirkland, Washington 98034 (US); Clement, Thomas J., Redmond, Washington 98052 (US); Intlekofer, Michael J., Bellevue, Washington 98006 (US)
(74) Representative: Andersson, Andy Rudy

(56) References cited:
- EP-A- 315 730
- EP-A- 0 219 568
- EP-A- 0 316 995
- DE-A- 3 806 458
- FR-A- 2 501 034
- US-A- 3 858 586

## Description

### BACKGROUND OF THE INVENTION

### Field of the Invention

The present invention is directed to an electrosurgical guidewire. More particularly, the invention is directed to a guidewire which is energized by radio frequency current to penetrate occlusive tissue so as to permit the subsequent passage of a therapeutic device for treatment.

### Description of the Prior Art

In the United States, heart disease, stroke and related disorders account for almost as many deaths as all other causes of death combined. The single largest cause of cardiovascular diseases is sclerosis -- a build-up of fatty or calcific deposits in the arterial lumen. These deposits can impair, and in severe cases, totally obstruct the flow of blood. A number of medical devices are designed to displace, disperse or extract the occlusive tissue. However, most of these devices operate over or in conjunction with a guidewire.

Initial placement of the guidewire is a problem in cases of total occlusion. Although some occlusions can be negotiated by forcible advancement of a blunt catheter (the Dotter technique), or by rotational means (orthogonal displacement of friction), a reliable method is required which will unconditionally traverse a total occlusion to permit guidewire insertion and subsequent passage of a therapeutic device for treatment.

It is well known that electrical currents are able to flow in tissue because of the presence of free electrons and ions in solution within the tissue structure. Many physiological functions are made possible by the electrical conductivity of the body. Passage of the electrical current in the mammalian or similar bodies dissipates some energy in the form of heat because of the finite electrical resistance of the body tissue (power density equalling resistivity "ohm-cm" times the square of current density (amps/cm²). At low current density values, this product of heat is totally insignificant. If the current density levels are increased, it is possible to vaporize the entrained tissue water. If this vaporization occurs with sufficient speed before the water vapor can diffuse through the tissue, it will locally rupture and incise tissue. Surgically producing such ruptures in a linear fashion is the essence of electrosurgery.

In order not to cause neuromuscular stimulation in electrosurgery, currents at frequencies in excess of 100 kilohertz are used. At these frequencies the muscle fibers are unable to respond. As electromagnetic radiation in this frequency range is used for the broadcast of radio signals, electrical currents in this frequency range are referred to as radio frequency, or RF. Electrosurgery is typically accomplished by passing RF current through a small electrode in the form of a scalpel or needle into the tissue and completing the electrical circuit through the tissue by attaching a much larger electrode plate (indifferent electrode or patient plate) elsewhere on the body. The small electrode in the form of a scalpel or needle is referred to as the active electrode because it is where the surgical effect occurs due to the much higher current density adjacent to the small area of the electrode. Since the total current at any place in the circuit is equal to the total current at any other place in the circuit at any instant of time, the current density (i.e., current divided by area) at the relatively large patient plate is extremely low, so as not to cause any noticeable effect on the patient. Conductive jellies are used to assure good conductance from the patient's body through the patient plate and to avoid hot spots and burns which would result from reduced current flow areas and high current densities.

Under certain circumstances, the patient plate or indifferent electrode can be eliminated. If the patient plate electrode circuit connected to the console line cord ground wire, the electrical energy can be coupled out of the patient's body capacitively to earth ground. Connecting the patient plate electrode circuit to the console line ground places the active electrode at high potential relative to the earth and the building, and thus the patient. The inconvenience of the patient plate is eliminated, and there is no risk of burns at the plate. Although electrosurgery can be performed using RF frequencies from 300 kilohertz to 3 megahertz, the stray capacitance technique benefits from the use of higher frequencies, since a stray capacitance at a given value would effect a lower impedance at a higher frequency. This is based upon the relationship whereby the reactance of a given capacitor is inversely proportional to frequency.

Electrosurgery using a single active electrode is properly defined as monopolar electrosurgery. Monopolar scalpels are commonly referred to as "Bovies" in honor of the person who popularized their use.

DE-A-3 806 458 (D3) discloses an arrangement as in the preamble of claim 1 wherein the conductive wire with its tip is fixed with respect to insulating sheet.

EP-A-0 315 730 discloses a device for expanding and/or opening blood vessels, having a conductive wire including a ballike end. Such conductive wire is to some extent adjustable.

### OBJECTS AND SUMMARY OF INVENTION

It is therefore an object of this invention to provide an apparatus for a guidewire to reliably traverse a total occlusion to act as a guide rail for the subsequent passage of a therapeutic device.

This and other objects of the invention are effectively attained by the instant invention which relates to the use of electrosurgical principles and technology to permit the passage of a guidewire through a totally occluded blood vessel. Specifically, the invention includes a slender conductive wire with a punctate tip on its distal end with insulation for a distance behind the tip, all within an insulative sheath. When the wire and sheath are introduced into the body using conventional angiographic techniques, the wire tip can be brought to bear against the occluding material in the vessel. When an RF current is passed through the wire to the occluding material, the occluding tissue adjacent to the tip is immediately electrosurgically ablated. The tip then makes rapid and unimpeded progress traversing the occlusion to allow subsequent passage of a therapeutic device for treatment.

### BRIEF DESCRIPTION OF THE DRAWINGS

Figure 1 discloses a perspective view of the present invention.

Figure 2 discloses a cross-sectional view of the distal end of the guidewire of the present invention.

Figure 3 discloses a perspective view of the Y-adapter of the present invention.

### DETAILED DESCRIPTION OF THE PREFERRED EMBODIMENT

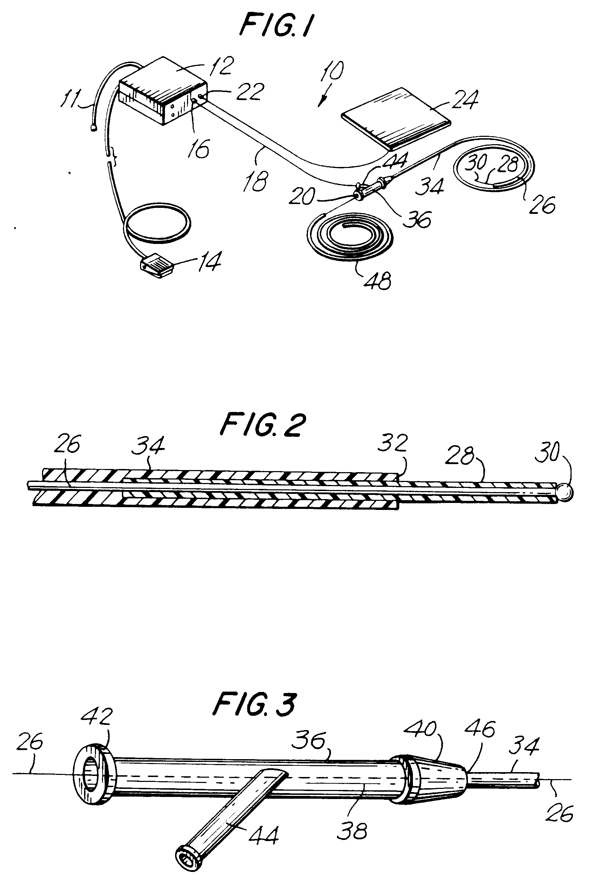

In reference to the drawings in detail wherein like numerals indicate like elements throughout the several views, Figure 1 discloses a perspective view of the present invention. Guidewire system 10 includes a radio frequency (RF) generator console 12 which receives power from a line cord 11 (which includes a ground wire) which plugs into a standard 110 volt grounded electrical socket (not shown). Radio frequency generator console 12 includes a switch means such as a foot pedal 14. A first terminal 16 of radio frequency generator 12 is attached to cable 18, which leads to electrical connection terminal 20 which is attached to the guidewire 26. A second terminal 22 of radio frequency generator 12 is attached to ground plate 24. Alternatively, the ground plate 24 can be eliminated, and the RF current can return to earth ground via the patient's intrinsic capacitance with respect to the universe. In this configuration, terminal 22 is internally connected to the ground wire of line cord 11.

Electrical connection terminal 20 fixedly engages and is electrically coupled to guidewire 26. It is within the scope of the invention that terminal 20 may be slidably connected to guidewire 26.

Guidewire 26 is relatively small, having a diameter of from about 0.101 mm to 0.762 mm (0.004 to 0.030 inches), preferably from about 0.177 to 0.508 mm (0.007 to 0.020 inches). A preferred guidewire 26 is preferably about 0.228 mm (0.009 inch) diameter 304 stainless steel to provide high strength and corrosion resistance. As shown in Figure 2, guidewire 26 has sufficient insulation 28, e.g., from about 12.7 to 152.4 mm or more (1/2 to 6 inches or more), here about 31.75 mm (1 1/4 inches, immediately behind distal tip 30 to prevent RF energy distribution from any point other than tip 30 of guidewire 26. The insulation 28 is preferably made of a suitable insulating material such as polytetrafluoroethylene (PTFE) (available as TEFLON ®from E.I. duPont) due to its low coefficient of friction, good electrical insulating properties, high coefficient of resistance, high operating temperature, and high dielectric strength.

Spheroidal distal tip 30 allows rapid progress into the blood vessel, while minimizing trauma or tendency to perforate. Although the tip 30 can be of any shape or size, the preferred embodiment is a spherical tip (referred to below as a "microball") of a diameter of from about 0.127 mm to 1.27 mm (0.005 to 0.050 inches), preferably from about 0.304 mm to 0.635 mm (0.012 to 0.025 inches). A tip of such small size produces little tissue injury and provides a small tissue channel which normally would clot if not enlarged with some other modality. In this way, even if arterial wall perforation occurred, the wall would normally clot owing to the tip's very small size. A further advantage of having a ball tip configuration is to reduce accidental perforation resulting from mechanical manipulation without electrical activation.

A spherical tip has the advantage of being easy to form on the guidewire 26 by arc welding techniques. Alternatively, the tip 30 may be formed of radiopaque material such as platinum to facilitate the monitoring of the position of the tip 30 by the use of X-rays. Tip 30 extends from opening 32 in guide sheath 34. Guidewire 26 passes through sheath 34 and is delivered to the electrosurgical site by use of standard angiographic techniques.

As shown in detail in Figure 3, the Y-adapter 36 includes a body 38 with a male Luer fitting 40 on a first end and a gland nut 42 on a second end. Guidewire 26 passes through both male Luer fitting 40 and gland nut 42. Sidearm 44 joins body 38 of Y-adapter 36 as an infusion port to allow the injection of contrast agents or saline solutions to be carried through sheath 34 and released at the distal end of sheath 34 (inside the patient), proximal to the tip 30.

Moreover, sheath 34 includes a female Luer fitting 46. The proximal end of guidewire 26 is threaded through male Luer fitting 40 of the Y-adapter 36 and thence through the sealing gland and gland nut 42. Feeding is continued until female Luer fitting 46 on sheath 34 is reached and male Luer fitting 40 of Y-adapter 36 is engaged thereto. Gland nut 42 on Y-adapter 36 is tightened to secure guidewire 26 in place relative to sheath 34. Relative positioning of the tip 30 of guidewire 26 to the opening 32 of sheath 34 is done by loosening gland nut 42, repositioning guidewire 26, and retightening gland nut 42. Any remaining portion of the proximal end of guidewire 26 extending from gland nut 42 some distance (electrical connection) terminal 20 passes into plastic tubing 48 to prevent inadvertant electrical pathways. The gland/gland nut 42 serves two purposes: 1) to secure the guidewire 26 in position relative to the sheath 34, and 2) to prevent the loss of fluid at the point where the wire exits the Y-adapter 36.

To use this invention, the ground plate 24 is attached to the patient using conductive jelly to ensure positive and dispersive electrical contact with the patient. Alternatively, the ground plate is dispensed with when the "stray capacitance" technique is used. With either technique, the guidewire 26 and sheath 34 are placed into the patient using standard angiographic techniques and the procedure described above to place the tip 30 proximally adjacent to the occlusive material in the artery (or other passageway or duct) in the patient.

The user depresses foot pedal 14 or other switch means to energize electrically the tip 30 via the guidewire 26, electrical connection terminal 20, cable 18, first terminal 16, and radio frequency (RF) generator console 12. Moderate pressure must be applied to the sheath 34 in the distal direction to cause the guidewire 26 and sheath 34 to advance in concert through an obstruction when energy is applied. It is important that constant longitudinal forward force be exerted during delivery of the electrosurgical current to avoid localized dehydration which would, in turn, cause high electrical resistivity which could impede further electro-ablation.

In a preferred embodiment for intravascular use, each depression of foot pedal 14 results in an energy pulse of approximately 3 joules of energy being delivered to the tissue contacting tip 30 of guidewire 26. The energy pulse has a duration of approximately 0.2 seconds and causes guidewire 26 to advance about an eighth of an inch in soft tissue. After the guidewire 26 has completely traversed the occlusion, the sheath 34 is removed, and the guidewire 26 is used to guide any of a variety of therapeutic devices (not shown) used to treat the occlusion or related disorders, including, but not limited to, percutaneous transluminal coronary angioplasty (PTCA) or percutaneous transluminal rotary ablation.

Thermal effects of the passage of the guidewire 26 are minimal since the delivered power density falls off inversely as the fourth power of the distance from the tip 30 (i.e., power density is proportional to the square of the current density, and current density is inversely proportional to the square of the distance from the tip 30) and due also to the small size of tip 30.

In a preferred embodiment, the output signal is a 120 hertz amplitude modulated, RF waveform with a peak voltage of about 600 volts into a 500 ohm load (about 1200 volts peak with no load). The 120 hertz modulation reduces the overall power delivered to the tissue. The high voltage provides rapid cutting under all circumstances, even submerged in saline.

Thus, the several aforementioned objects and advantages are most effectively attained. Although preferred embodiments of the invention have been disclosed and described in detail herein, it should be understood that this invention is in no sense limited thereby and its scope is to be determined by that of the appended claims.

## Claims

1. A catheter system for ablating and penetrating occlusive tissue to open a substantially occluded arterial lumen and for minimizing scarring surrounding arterial tissue, comprising
a substantially tubular insulating sheath having proximal and distal ends and having a lumen extending therethrough;
an electrically conductive guidewire (26), wherein said guidewire has a proximal end and an electrosurgical distal tip (30); and
a voltage generating means (12) electrically connected to said electrically conductive guidewire to activate said electrosurgical distal tip in monopolar fashion,
**characterized** in that said distal tip is punctate and extends slidably through said lumen such that the tubular sheath (34) is an insulator for said guidewire, wherein said guidewire has an insulative coating (28) proximally adjacent to the electrosurgical distal tip, wherein said distal tip has a diameter of from 0.127 mm to 1.27 mm (0.005 inches to 0.05 inches) and wherein said voltage generating means generates an alternating current voltage.

2. The catheter system of Claim 1, wherein said voltage generating means includes a first and a second terminal, said first terminal is electrically connected to said guidewire, and said second terminal is electrically connected to a ground plate for attachment to a patient.

3. The catheter system of Claim 1, wherein said voltage generating means incudes a first and a second terminal, said first terminal is electrically corrected to said guidewire, and said second terminal is electrically connected to a power supply ground wire, wherein a patient and said supply ground wire are connected via capacitive coupling.

4. The catheter system of Claim 1, wherein said alternating current voltage has a frequency in excess of 100 kHz.

5. The catheter system of Claim 1, wherein said alternating current voltage has a frequency in the radio-frequency range.

6. The catheter system of Claim 1, wherein said alternating current voltage is a 120 hertz amplitude modulated, RF waveform with a peak voltage of about 600 volts into a 500 ohm load and about 1200 volts peak with no load.

7. The catheter system of Claim 1, wherein said guidewire distal tip is a microball.

8. The catheter system of Claim 1, which also comprises an adapter engaging the proximal end of the sheath, which adapter has means for releasably securing said guidewire into a range of positions.

9. The catheter system of Claim 8 wherein the proximal end of the guidewire is circumferentially surrounded by an insulative means which prevents inadvertent electrical pathways.

10. The catheter system of Claim 8, wherein said means for releasably securing said guidewire includes a gland and gland nut.

11. The catheter system of Claim 8, wherein the proximal end of said sheath has a Luer fitting which engages a reciprocal Luer fitting on said adapter.

12. The catheter system of Claim 8, wherein said adapter includes an injection port of communication with said sheath.

13. The catheter system of Claim 8, wherein said means for releasably securing said guidewire has (1) a gland and gland nut and wherein said adapter includes an insulated passage into which the proximal end of said guidewire passes and (2) a Luer fitting to engage said sheath.

14. The catheter system of Claim 1 wherein said voltage generating means generates an alternating current voltage with a frequency substantially equal to 500 kHz.

15. The catheter system of Claim 1, wherein said voltage generating means generates an alternating current voltage that is amplitude modulated at a frequency of 50 to 200 Hz.

16. The catheter system of Claim 1, wherein said voltage generating means is operatively connected to a switch means which operates the voltage generating means for a predetermined period.

17. The catheter system of Claim 16, wherein said predetermined period os approximately 0.2 seconds.

18. The catheter system of Claim 16, wherein said switch means comprises a foot pedal.

## Patentansprüche

1. Kathetersystem zum Abtragen und Durchdringen eines Verschlußgewebes, um einen im wesentlichen verschlossenen arteriellen Hohlraum zu öffnen und eine Vernarbung des umgebenden arteriellen Gewebes zu minimieren, umfassend
einen im wesentlichen rohrförmigen isolierenden Mantel, der vordere und hintere Enden und einen sich durch diesen erstreckenden Hohlraum hat;
einen elektrisch leitfähigen Führungsdraht (26), wobei der Führungsdraht ein Hinterende und ein elektrooperatives Vorderende (30) hat; und einen Spannungserzeuger (12), der mit dem elektrisch leitfähigen Führungs
draht elektrisch verbunden ist, um das elektrooperative Vorderende auf einpolige Weise zu aktivieren,
dadurch **gekennzeichnet,** daß das Vorderende punktförmig ist und sich verschiebbar durch den Hohlraum erstreckt, so daß der rohrförmige Mantel (34) eine Isolierung für den Führungsdraht ist, wobei der Führungsdraht eine isolierende Beschichtung (28) nahe dem elektrooperativen Vorderende hat, wobei dieses Vorderende einen Durchmesser von 0,127 mm bis 1,27 mm (0,005 inches bis 0,05 inches) hat und wobei der Spannungserzeuger eine Wechselspannung erzeugt.

2. Kathetersystem nach Anspruch 1, wobei der Spannungserzeuger einen ersten und einen zweiten Anschluß hat, wobei der erste Anschluß mit dem Führungsdraht elektrisch verbunden ist und der zweite Anschluß mit einer Erdungsplatte zur Befestigung an einem Patienten elektrisch verbunden ist.

3. Kathetersystem nach Anspruch 1, wobei der Spannungserzeuger einen ersten und einen zweiten Anschluß hat, wobei der erste Anschluß mit dem Führungsdraht elektrisch verbunden ist und der zweite Anschluß mit einem Leistungs-Erdungsdraht elektrisch verbunden ist, wobei ein Patient und der Leistungs-Erdungsdraht über eine kapazitive Kopplung verbunden sind.

4. Kathetersystem nach Anspruch 1, wobei die Wechselstromspannung eine Frequenz von mehr als 100 kHz hat.

5. Kathetersystem nach Anspruch 1, wobei die Wechselstromspannung eine Frequenz im Bereich der Radiofrequenz hat.

6. Kathetersystem nach Anspruch 1, wobei die Wechselstromspannung eine 120 Hz amplitudenmodulierte Radiofrequenz Wellenform mit einer Spitzenspannung von ungefähr 600 V bei einem Widerstand von 500 Ω und einer Spitzenspannung von ungefähr 1200 V ohne Widerstand hat.

7. Kathetersystem nach Anspruch 1, wobei das Vorderende des Führungsdrahtes eine Mikrokugel ist.

8. Kathetersystem nach Anspruch 1, ferner umfassend ein das Hinterende des Mantels erfassendes Verbindungsstück, das eine Einrichtung aufweist, um den Führungsdraht in mehreren Positionen lösbar festzulegen.

9. Kathetersystem nach Anspruch 8, wobei das Hinterende des Führungsdrahtes am Umfang von einer isolierenden Einrichtung umgeben ist, die Kriechströme verhindert.

10. Kathetersystem nach Anspruch 8, wobei die Einrichtung zum lösbaren Festlegen des Führungsdrahtes eine Stopfbüchse und eine Überwurfmutter umfaßt.

11. Kathetersystem nach Anspruch 8, wobei das Hinterende des Mantels einen Luer-Anschluß aufweist, der mit einem entsprechenden Luer-Anschluß an dem Verbindungsstück in Eingriff steht.

12. Kathetersystem nach Anspruch 8, wobei das Verbindungsstück eine mit dem Mantel in Verbindung stehende Injektionsöffnung aufweist.

13. Kathetersystem nach Anspruch 8, wobei die Einrichtung zum lösbaren Festlegen des Führungsdrahtes (i) eine Stopfbüchse und eine Überwurfmutter aufweist, und wobei das Verbindungsstück einen isolierten Durchgang hat, in den sich das Hinterende des Führungsdrahtes erstreckt, und (ii) einen Luer-Anschluß aufweist, um den Mantel zu erfassen.

14. Kathetersystem nach Anspruch 1, wobei der Spannungserzeuger eine Wechselstromspannung mit einer Frequenz von näherungsweise 500 kHz erzeugt.

15. Kathetersystem nach Anspruch 1, wobei der Spannungserzeuger eine Wechselstromspannung erzeugt, die mit einer Frequenz von 50 bis 200 Hz amplitudenmoduliert ist.

16. Kathetersystem nach Anspruch 1, wobei der Spannungserzeuger mit einer Schalteinrichtung gekuppelt ist, die den Spannungserzeuger für eine bestimmte Zeitdauer einschaltet.

17. Kathetersystem nach Anspruch 16, wobei die bestimmte Zeitdauer ungefähr 0,2 Sekunden beträgt.

18. Kathetersystem nach Anspruch 16, wobei die Schalteinrichtung ein Fußpedal umfaßt.

## Revendications

1. Système de cathéter destiné à réaliser une ablation d'un tissu occlusif et à pénétrer un tissu occlusif pour ouvrir une ouverture artérielle sensiblement occlusée et destiné à minimiser le tissu artériel environnant ayant subit une piqûre, comportant
une gaine isolante sensiblement tubulaire ayant des extrémités proximale et distale et ayant une ouverture la traversant;
un fil (26) guide conducteur électriquement, le fil guide ayant une extrémité proximale et une pointe (30) distale électrochirurgicale; et
des moyens (12) de production de tension reliés électriquement au fil guide conducteur électriquement pour activer la pointe distale électrochirurgicale d'une manière monopolaire,
caractérisé en ce que la pointe distale est ponctuée et s'étend de manière coulissante dans l'ouverture de sorte que la gaine (34) tubulaire est un isolant pour le fil guide, le fil guide ayant un revêtement (28) isolant adjacent proximalement à la pointe distale électrochirurgicale, la pointe distale ayant un diamètre compris entre 0,127 mm à 1,27 mm (0,005 pouce et 0,05 pouce) et les moyens de production de tension produisant une tension de courant alternatif.

2. Système de cathéter suivant la revendication 1, dans lequel les moyens de production de tension comportent une première borne et une seconde borne, la première borne est connectée électriquement au fil guide, et la seconde borne est connectée électriquement à une plaque de terre pour la fixation à un patient.

3. Système de cathéter suivant la revendication 1, dans lequel les moyens de production de tension comportent une première et une seconde borne, la première borne étant connectée électriquement au fil guide et la seconde borne étant connectée électriquement à un fil de terre d'alimentation en puissance dans lequel un patient et le fil de terre d'alimentation sont reliés par l'intermédiaire d'un couplage capacitif.

4. Système de cathéter suivant la revendication 1, dans lequel la tension de courant alternatif a une fréquence supérieure à 100kHz.

5. Système de cathéter suivant la revendication 1, dans lequel la tension de courant alternatif a une fréquence dans la gamme de radio fréquence.

6. Système de cathéter suivant la revendication 1, dans lequel la tension du courant alternatif est une forme d'onde RF modulée en amplitude à 120 hertz ayant une tension pic d'environ 600 volts dans une charge de 500 ohm et une tension pic d'environ 1200 volts sans charge.

7. Système de cathéter suivant la revendication 1, dans lequel la pointe distale de fil guide est une microballe.

8. Système de cathéter suivant la revendication 1, qui comporte également un adaptateur coopérant avec l'extrémité proximale de la gaine, cet adaptateur comportant des moyens destinés à fixer de manière amovible le fil guide dans une gamme de positions.

9. Système de cathéter suivant la revendication 8, dans lequel l'extrémité proximale du fil guide est entourée de manière circonférentielle par des moyens isolants qui empêchent l'existence de trajets électriques apparaissant par inadvertance.

10. Système de cathéter suivant la revendication 8, dans lequel les moyens destinés à fixer de manière amovible le fil guide comportent une presse-étoupe et un écrou presse-étoupe.

11. Système de cathéter suivant la revendication 8, dans lequel l'extrémité proximale de la gaine a un raccord Luer qui coopère avec un raccord Luer réciproque sur l'adaptateur.

12. Système de cathéter suivant la revendication 8, dans lequel l'adaptateur comporte un orifice d'injection de communication avec la gaine.

13. Système de cathéter suivant la revendication 8, dans lequel les moyens destinés à fixer de manière amovible le fil guide comportent (1) une presse-étoupe et un écrou presse-étoupe et dans lequel l'adaptateur comporte un passage isolant dans lequel l'extrémité proximale du fil guide passe et (2) un raccord Luer qui coopère avec la gaine.

14. Système de cathéter suivant la revendication 1, dans lequel les moyens de production de tension produisent une tension de courant alternatif ayant une fréquence sensiblement égal à 500kHz.

15. Système de cathéter suivant la revendication 1, dans lequel les moyens de production de tension produisent une tension de courant alternatif qui est modulée en amplitude à une fréquence de 50 à 200 Hz.

16. Système de cathéter suivant la revendication 1, dans lequel les moyens de production de tension sont connectés de manière fonctionnel à des moyens de commutation qui font fonctionner les moyens de production de tension pour une période prédéterminée.

17. Système de cathéter suivant la revendication 16, dans lequel la période prédéterminée est d'approximativement 0,2 seconde.

18. Système de cathéter suivant la revendication 16, dans lequel les moyens de commutation comportent une pédale à pied.
